# EUROPEAN PATENT SPECIFICATION

(11) **EP 1 701 726 B1**
(45) Date of publication and mention of the grant of the patent: **23.01.2019**
(21) Application number: 04782618.5
(22) Date of filing: 30.08.2004
(51) Int. Cl.: A61K 31/495, A61K 31/50, A61P 31/04

(54) **Composition for use in photodynamic therapy**
Stoffgemisch zur Anwendung in der photodynamischen Therapie
Composition pour utilisation dans la thérapie photodynamique

(30) Priority: 02.09.2003 US 499847 P; 03.06.2004 US 860296
(43) Date of publication of application: 20.09.2006
(73) Proprietor: biolitec Unternehmensbeteiligungs II AG, 1030 Vienna (AT)
(72) Inventor: ALBRECHT, Volker, D-14558 Nuthetal, OT Bergholz-Rehbrücke (DE); GITTER, Burkhard, D-07743 Jena (DE)
(74) Representative: Herrmann, Franz
(86) International application number: PCT/US2004/028180
(87) International publication number: WO 2005/021094

(56) References cited:
- WO-A2-2005/034855
- US-A1- 2003 059 379
- US-B2- 6 506 791
- DATABASE CA [Online] CHEMICAL ABSTRACTS SERVICE, COLUMBUS, OHIO, US; T'UNG, T.: "Photodynamic action of safranine on Gram-negative bacilli" XP002408496 retrieved from STN Database accession no. 1939:51245 & PROCEEDINGS OF THE SOCIETY FOR EXPERIMENTAL BIOLOGY AND MEDICINE , 39, 415-17 CODEN: PSEBAA; ISSN: 0037-9727, 1938,
- DATABASE CA [Online] CHEMICAL ABSTRACTS SERVICE, COLUMBUS, OHIO, US; YAMAMOTO, NOBUTO: "Photodynamic action on bacteriophage. I. The relation between the chemical structure and photodynamic activity of various dyes" XP002408497 retrieved from STN Database accession no. 1957:72192 & UIRUSU , 6, 510-21 CODEN: UIRUAF; ISSN: 0042-6857, 1956,
- SACHDEVA RUCHI ET AL: "Dye mediated photoinactivation of bacteriophages by nitrogen laser" JOURNAL OF BIOSCIENCES (BANGALORE), vol. 24, no. 1, March 1999 (1999-03), pages 79-83, XP008071778 ISSN: 0250-5991
- T'UNG T: "Photodynamic action of safranine on Gram-negative bacilli", PROCEEDINGS OF THE SOCIETY FOR EXPERIMENTAL BIOLOGY & MEDICINE, ACADEMIC PRESS INC. NEW YORK, US, vol. 39, 1 January 1938 (1938-01-01), pages 415-417, XP008089432, ISSN: 0037-9727

## Description

### Background of the Invention

### 1. Field of the invention

The invention relates to the field of photodynamic therapy, particularly the use of photodynamic therapy in the selective destruction of bacteria in human and animal patients.

### 2. Information Disclosure Statement

Photodynamic therapy (PDT) is well known and has been utilized to combat numerous diseases generally associated with hyperproliferating tissue, such as cancer and various skin conditions. PDT has also been utilized as an antimicrobial treatment. However, there are two major problems associated with antimicrobial PDT. The first problem stems from the difficulty of finding photoactive substances that can be effectively used against both Gram-positive and Gram-negative bacteria. Gram-negative bacteria present a much tougher obstacle primarily due to their double-layer outer membrane structure.

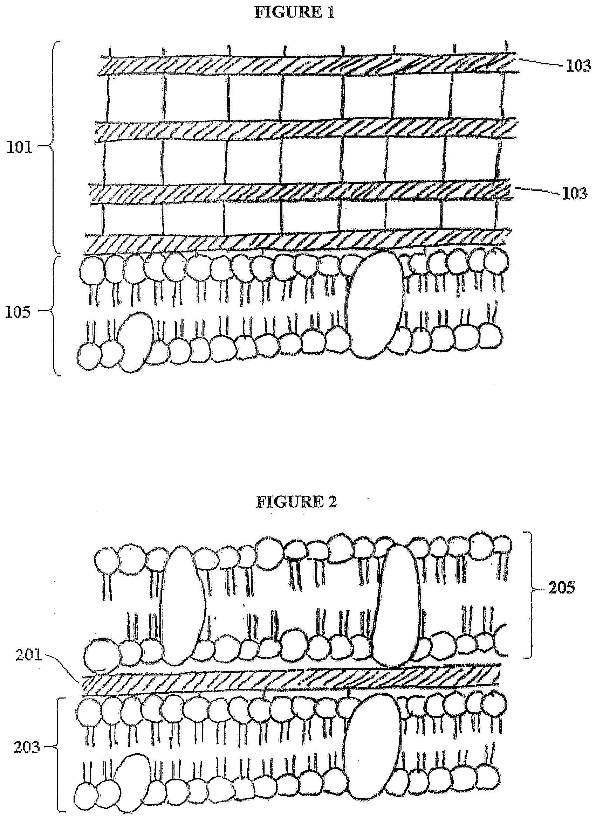

The primary difference between Gram-positive and Gram-negative bacteria lies in the cell walls, as is illustrated in **Figures 1** and **2**. As shown in **Figure 1**, Gram-positive cells have a thick peptidoglycan cell wall **101**, consisting of many individual peptidoglycan layers **103** (for example, 20-40 layers) surrounding cell membrane **105**. In contrast, as shown in figure **2**, Gram-negative cells have only a thin layer of peptidoglycan **201** surrounding cell membrane **203**, which is further surrounded by an additional outer membrane **205**. This additional layer allows Gram-negative and Gram-positive bacteria to be differentiated using Gram's method. Because of the outer membrane in Gram-negative bacteria, the crystal violet-iodine stain cannot reach the peptidoglycan layer of the cell wall and be retained in Gram-negative bacteria after Gram's method as it is in Gram-positive bacteria. The outer membrane is primarily responsible for inhibiting penetration of many substances into Gram-negative bacteria, and is the reason for the difficulty in finding photosensitizers that are effective against both types of bacteria.

The second problem results from difficulty in finding a suitable photosensitive compound that retains at least some activity in the presence of complex media such as blood serum, blood or saliva. Most photosensitive compounds (photosensitizers) that display good activity against cell suspensions in poor media such as phosphate buffered saline show virtually no effect in the presence of blood serum, blood or saliva. This is the case because the components in these complex media (e.g. proteins, blood cells) compete with the bacteria for affinity of the PDT compound.

Safranin O is a red dye that absorbs in the 450-600 nm blue-green range. It is used as a biological stain in processes such as photomicrography. For example, Safranin O stains nuclei, chromosomes, lignified and cutinized cell walls red. It is also used in conjunction with Gram's iodine for differentiating bacteria, and is used in electron microscopy. Safranine products have also been utilized in ink compositions used to indicate conditions involving time, pressure, energy, or the presence/absence of certain chemicals in sterilization techniques. (U.S. Patent No. 5,990,199)

T'ung discloses the photodynamic action of safranine on gram-negative bacteria (*Proc. Soc. Exp. Biol. Med.,* **39**, 415 (1938)).

Safranin O, safranine and related dyes have been used in periodontal treatments which detect and treat microbes and cavities on and around the teeth and gums. U.S. Patent No. 6,337,357 discloses an antimicrobial caries-detecting composition comprising water, a water-miscible solvent or a combination, a dye capable of staining the caries-infected portions of teeth, and an antimicrobial agent. This is both a cavity detection and sterilization system. A dye such as Safranin O, which is among the suitable dyes that are soluble in the solvent or solvents and capable of visually indicating the presence and location of cavities, may be used. For this invention, safranine is utilized purely as a staining agent and is not contemplated as an antimicrobial agent.

Safranin has also been used as a photosensitizer in antimicrobial PDT applications. U.S. Patent No. 6,558,653 (and U.S. Patent Application No. 2003/0059379) describes a method of PDT and photosensitive compounds for destroying necrotic tissue and bacteria on teeth. The method involves applying a dye composition, such as safranin, that absorbs light energy of a wavelength between 450 and 600 nm. The dye contacts the necrotic tissue, bacteria and surrounding tissue to selectively stain the tissue. The stained infected tissue thus readily absorbs the applied radiation and thermally destroys the diseased tissue and bacteria. This invention is limited to the treatment of surface bacteria in the periodontal pocket using photothermal effects.

The photoactive characteristics of Safranin O have been utilized in other applications, such as insecticidal and non-human antimicrobial treatments and compositions. U.S. Patent No. 5,798,112 describes the use of photoactive dyes such as Safranin O in a phototoxic insecticidal composition. The composition contains selected photoactive dyes, a bait, and an adjuvant. The compound is ingested by desired insects, whereby the adjuvant interacts with the photoactive dye and the insect membranes to alter the toxicity of the composition, which acts to kill the insects after exposure to sunlight for a period of time. U.S. Patent No. 6,506,791 discloses a method of treating protozoan infections in fish. A photoactive dye including Safranin O is introduced into an aqueous environment containing infected fish, such that the concentration of the photoactive dyes is sufficient to kill some or all of the bacteria.

There is a need for an antimicrobial PDT method and compound which is effective in the presence of complex media such as blood serum, blood or saliva. This method should be effectively used against both Gram-positive and Gram-negative bacteria. The present invention addresses this need.

### Objectives and Brief Summary of the Invention

It is an object of the present invention to provide a compound for use in a method for the efficient and selective destruction of harmful microbes, especially bacteria, in a human or animal subject.

It is another object of the present invention to provide a compound for use in an anti-bacterial method that can be controllably and selectively activated by electromagnetic radiation.

It is yet another object of the present invention to provide a compound for use in a method that is effective for the destruction of both Gram-positive and Gram-negative bacteria.

Briefly stated, the present invention provides a composition for use in destroying bacteria, in the body utilizing Safranin O in conjunction with electromagnetic radiation. A composition comprising Safranin O is introduced to a treatment area. After a sufficient period of time has elapsed, radiation of a suitable wavelength is applied to the area to activate the Safranin O and by a photodynamic reaction to destroy the bacteria. The radiation has a wavelength between 500 nm and 580 nm. The compound for use in a method is effective for destroying both Gram-positive and Gram-negative bacteria in areas where blood serum, plasma, blood or saliva are also present.

The above, and other objects, features and advantages of the present invention will become apparent from the following description read in conjunction with the accompanying drawings.

### Brief Description of Figures

Fig. 1 - Cross-sectional view of the cell envelope of a Gram-positive bacteria cell.
Fig. 2 - Cross-sectional view of the cell envelope of a Gram-negative bacteria cell.
Fig. 3 - Graph showing photodynamic inactivation of *Staphylococcus aureus DSM1104* by Safranin O.
Fig. 4 - Graph showing photodynamic inactivation of *Pseudomonas aeruginosa DSM1117* by Safranin O.
Fig. 5 - Graph showing photodynamic inactivation of *Escherichia coli DSM8698* by Safranin O.

### Detailed Description of Preferred Embodiments

Because of the difficulties found in prior art methods and compounds, particularly in providing ways to destroy both Gram-positive and Gram-negative bacteria and avoid the deleterious effects of complex media such as blood serum, blood or saliva, it is desirable to find a compound that overcomes these disadvantages. Surprisingly, it was discovered that Safranin O can be used effectively to destroy both Gram-positive as well as Gram-negative germs in conjunction with electromagnetic radiation. Another advantage noted was that the presence of complex components of the medium (e.g. blood serum, blood or saliva) does not neutralize the effectiveness of Safranin O in targeting bacteria, as is often the case with other photosensitizers. Safranin O is thus part of an effective anti-bacterial treatment according to the present invention.

The antibacterial treatment contains three general steps. The first step is to introduce the Safranin O composition to an environment containing bacteria. The second step is to allow a sufficient period of time to elapse to allow the Safranin O to penetrate into the bacteria cells in the treatment area or at least bind onto components of their cell envelope. The final step is to apply radiation of a suitable wavelength to initiate a photodynamic mechanism by activation of Safranin O causing the production of reactive oxygen species and free radicals leading to the destruction of the bacteria.

The period of time between application of the Safranin O composition and irradiation is at least 15 minutes. For treating internal bacterial infections, the composition may be injected into the bloodstream for systemic application, or locally injected if the infection is confined to a specific area. For infections on or near the skin, the composition may be in the form of a solution, cream, gel or lotion for topical application. After the preselected period of time, radiation is applied to the treatment site to activate the Safranin O and destroy bacteria. The wavelength of the activating radiation is between 500 nm and 580 nm, and is even more preferably 530 nm. The radiation can be non-coherent radiation such as from a lamp, or coherent laser radiation. For surface or subsurface treatments, a lamp may be effective in irradiating specific infected areas, whereas for infected areas deeper within the body, an optical fiber apparatus, including one or more optical fibers, which may further contain diffusers or other devices as needed to irradiate a certain internal area, is preferred to deliver laser radiation to those internal areas. A preferred laser source is a diode pumped 532 nm laser

The present invention is further illustrated by the following examples.

### Example 1:

### Photodynamic inactivation of bacterial cell suspensions by Safranin O:

Several studies have demonstrated that Gram-positive bacteria (e.g. *Staphylococcus aureus*) are particularly susceptible to photodynamic inactivation whereas Gram-negative bacteria (e.g. *Escherichia coli, Pseudomonas aeruginosa)* are significantly more resistant to many commonly used photosensitizers. Moreover, we found that both Gram-positive and Gram-negative bacterial cells in more complex media (e.g. blood, plasma, blood serum, saliva) are also more protected from photodynamic action.

The organisms used in our studies were three members of the microflora of wounds: *Staphylococcus aureus DSM1104 (ATCC 25923),* Gram-positive; *Escherichia coli DSM 8698,* Gram-negative; *Pseudomonas aeruginosa DSM 1117* (*ATCC 27853*), Gram-negative. All strains were grown aerobically overnight at 37 °C in Tryptic Soy Broth (Merck KGaA Darmstadt, Germany). Cells were harvested by centrifugation and resuspended in sterile phosphate-buffered saline (PBS) or sterile PBS supplemented with 10 % sterile horse blood serum (Oxoid), 10 % human plasma or 10 % human blood (both freshly obtained from donors), respectively. The final OD (Optical Density) at 600 nm, 1 cm in all cases was 0.03.

Aliquots (190 µl) of the bacterial suspensions were placed into sterile black 96 well plates with clear bottom (Costar® 3603, Corning Inc., USA). 10 µl of three different Safranin O stock solutions were added to obtain the final photosensitizer concentrations of 10 µM, 100 µM and 1 mM, respectively. The plates were incubated for 30 minutes in the dark at room temperature.

After incubation the samples were exposed to light from a laser Ceralas G2 (biolitec AG, Germany), 532 nm, power set to 0.5 W, irradiation time of 85 s via a light fiber from the bottom of the plate. The fluence rate for these settings was about 1.2 W/cm² (measured with Optometer P-9710, Gigahertz-Optik GmbH, Puchheim, Germany). With the irradiation time, the resulting energy fluence was about 100 J/cm².

Control wells contained no Safranin O and were not exposed to laser light. The control samples for dark toxicity were only exposed to Safranin O (end concentration of 1 mM) without any illumination.

After illumination the samples were removed from the wells of the plate, diluted with Tryptic Soy Broth and plated by using spiral plater Eddy Jet (iul Instruments, Barcelona, Spain) on Tryptic Soy agar plates. The numbers of colony-forming units (CFU/ml) were enumerated after adequate incubation by using colony counter Countermat Flash (iul Instruments, Barcelona, Spain).

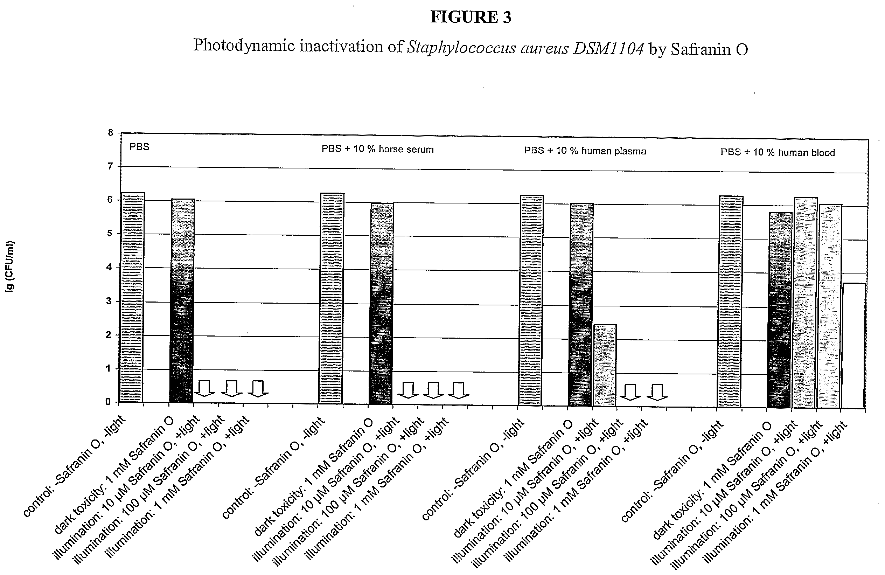

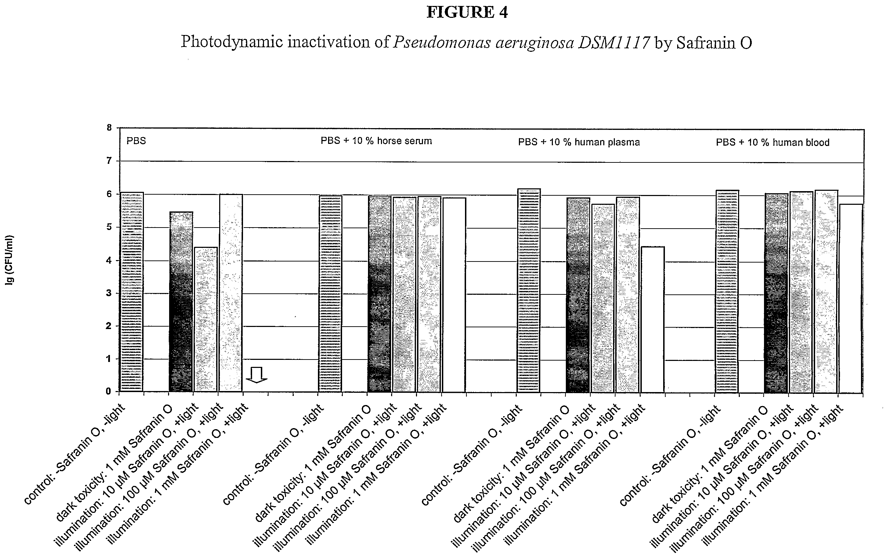

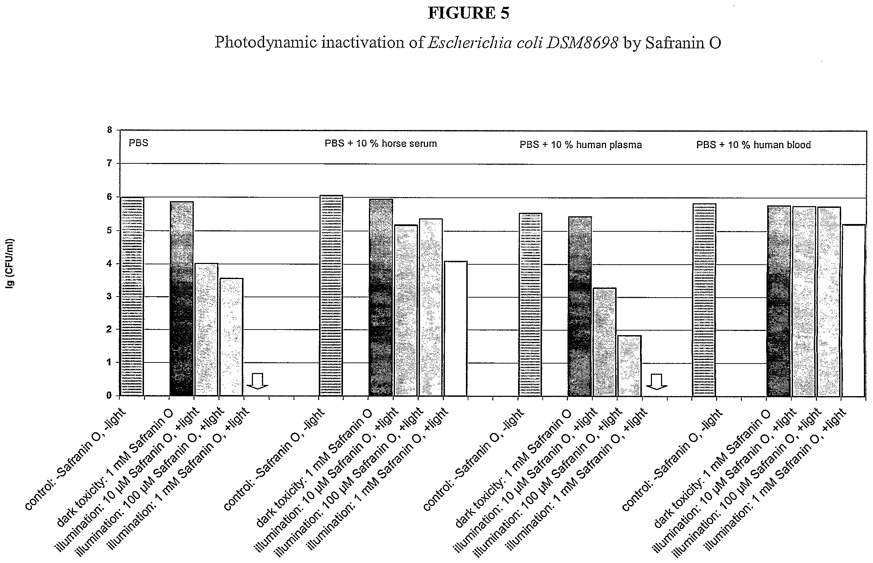

The results of the experiments are shown in Figures 3, 4 and 5: We found a very good killing effect by PDT treatment with Safranin O in the case of suspensions of Gram-positive *Staphylococcus aureus* cells in PBS and in more complex media (PBS + 10 % horse blood serum, PBS + 10 % human plasma; see Figure 4). Moreover there was also a sufficient killing of cell suspensions of Gram-negative *Pseudomonas aeruginosa* and *Escherichia coli* cells in presence of blood serum or plasma in the treatment suspensions (see Figures 4 and 5, respectively).

## Claims

1. A composition comprising Safranin O for use in destroying bacteria in humans by application of radiation of a preselected wavelength to activate Safranin O following introduction to a treatment area, where a blood serum, blood plasma, blood or saliva is present, and allowing a predetermined time period to elapse to allow said Safranin O to couple with bacteria in said treatment area and stimulate a photodynamic reaction to destroy said bacteria,
wherein said preselected wavelength is between 500 nm and 580 nm, and wherein said predetermined time period is at least 15 minutes.

2. The composition according to claim 1,
wherein said preselected wavelength is 530 nm.

3. The composition according to claim 1 or 2,
wherein said composition is introduced to a treatment area by one of systemic application, local application and topical application.

4. The composition according to claim 3,
wherein said systemic application is intravenous injection.

5. The composition according to claim 3,
wherein said local application is local injection into non-vascular tissue.

6. The composition according to claim 3,
wherein said composition is in a form selected from the group consisting of a solution, a cream, a gel and a lotion for topical application.

7. The composition according to any one of claims 1 to 6, wherein that said radiation is provided by a radiation source selected from the group consisting of non-coherent lamps and coherent lasers.

8. The composition according to any one of claims 1 to 7, wherein the application of radiation of a preselected wavelength is accomplished by at least one optical fiber coupled to a radiation source.

## Patentansprüche

1. Zusammensetzung, die Safranin O aufweist, zur Verwendung bei der Zerstörung von Bakterien in Menschen durch die Anwendung von Strahlung einer vorbestimmten Wellenlänge, um Safranin O zu aktivieren, nach der Einführung in einem Behandlungsbereich, in dem Blutserum, Blutplasma, Blut oder Speichel vorhanden ist, und durch Zulassen des Verstreichens einer vorbestimmten Zeitspanne, um Safranin O zu gestatten, an Bakterien in dem Behandlungsbereich anzukoppeln und eine photodynamische Reaktion in Gang zu setzen, um die Bakterien zu zerstören,
wobei die vorbestimmte Wellenlänge zwischen 500 nm und 580 nm liegt, und
wobei die vorbestimmte Zeitspanne wenigstens 15 Minuten beträgt.

2. Zusammensetzung nach Anspruch 1,
bei der die vorbestimmte Wellenlänge 530 nm ist.

3. Zusammensetzung nach Anspruch 1 oder 2,
bei der die Zusammensetzung in den Behandlungsbereich durch eine systemische Anwendung, eine lokale Anwendung oder eine topische Anwendung eingebracht wird.

4. Zusammensetzung nach Anspruch 3,
bei der die systemische Anwendung eine intravenöse Injektion ist.

5. Zusammensetzung nach Anspruch 3,
bei der die lokale Anwendung eine lokale Injektion in nichtvaskuläres Gewebe ist.

6. Zusammensetzung nach Anspruch 3,
bei der die Zusammensetzung in einer Form vorliegt, die aus der Gruppe ausgewählt ist, die aus einer Lösung, einer Creme, einem Gel oder einer Lotion für topische Anwendungen ausgewählt ist.

7. Zusammensetzung nach einem der Ansprüche 1 bis 6,
bei der die Strahlung durch eine Strahlungsquelle bereitgestellt wird, die aus der Gruppe ausgewählt ist, die aus nicht-kohärenten Lampen und kohärenten Lasern besteht.

8. Zusammensetzung nach einem der Ansprüche 1 bis 7,
bei der die Anwendung einer Strahlung einer vorbestimmten Wellenlänge durch wenigstens eine an eine Strahlungsquelle angekoppelte optische Faser bewerkstelligt ist.

## Revendications

1. Composition comprenant une Safranine O destinée à être utilisée dans la destruction de bactéries chez l'homme par application de rayonnement d'une longueur d'onde présélectionnée pour activer la Safranine O suite à l'introduction vers une zone de traitement, où un sérum sanguin, plasma sanguin, du sang ou de la salive est présent, et laisser une période de temps prédéterminée s'écouler pour permettre à ladite Safranine O de se coupler à des bactéries dans ladite zone de traitement et stimuler une réaction photodynamique pour détruire lesdites bactéries,
dans laquelle ladite longueur d'onde présélectionnée est comprise entre 500 nm et 580 nm, et
dans laquelle ladite période de temps prédéterminée est d'au moins 15 minutes.

2. Composition selon la revendication 1,
dans laquelle ladite longueur d'onde présélectionnée est de 530 nm.

3. Composition selon la revendication 1 ou 2,
dans laquelle ladite composition est introduite vers une zone de traitement par une application parmi une application systémique, application locale et application topique.

4. Composition selon la revendication 3,
dans laquelle ladite application systémique est une injection intraveineuse.

5. Composition selon la revendication 3,
dans laquelle ladite application locale est une injection locale dans du tissu non vasculaire.

6. Composition selon la revendication 3,
dans laquelle ladite composition est sous une forme sélectionnée parmi le groupe constitué d'une solution, d'une crème, d'un gel et d'une lotion pour application topique.

7. Composition selon l'une quelconque des revendications 1 à 6,
dans laquelle ledit rayonnement est fourni par une source de rayonnement sélectionnée parmi le groupe constitué de lampes non cohérentes et de laser cohérents.

8. Composition selon l'une quelconque des revendications 1 à 7,
dans laquelle l'application de rayonnement d'une longueur d'onde présélectionnée est réalisée par au moins une fibre optique couplée à une source de rayonnement.
